# EUROPEAN PATENT APPLICATION

(11) **EP 4 851 654 A1**
(43) Date of publication of application: **09.12.2026**
(21) Application number: 26182988.1
(22) Date of filing: 03.06.2026
(51) Int. Cl.: G01R 33/36, A61B 5/00, G01R 33/28

(54) **MAGNETIC RESONANCE IMAGING SUPPORT APPARATUS, OPERATION METHOD OF MAGNETIC RESONANCE IMAGING SUPPORT APPARATUS, AND PROGRAM**

(30) Priority: 05.06.2025 JP 2025094387
(71) Applicant: FUJIFILM Corporation, Tokyo 106-8620 (JP)
(72) Inventor: HAMADA, Kota, Tokyo, 1068620 (JP)
(74) Representative: Dehns Germany Partnerschaft mbB

(57) **Abstract**

Provided are a magnetic resonance imaging support apparatus (200), an operation method of a magnetic resonance imaging support apparatus (200), and a program that present a recommended combination of a receive coil (110), a fixing tool (122), and a mat (124) to an examiner. A magnetic resonance imaging support apparatus (200) includes a processor (202), in which the processor (202) is configured to acquire examination information (220) including an examination site of a subject (patient) (9) to be imaged by a magnetic resonance imaging apparatus (MR imaging apparatus) (100), input the examination information (220) to a learning model (224), acquire a combination corresponding to the examination information (220) from the learning model (224), and output the combination as presentation information (300).

## Description

### BACKGROUND OF THE INVENTION

### 1. Field of the Invention

The present invention relates to a magnetic resonance imaging support apparatus, an operation method of the magnetic resonance imaging support apparatus, and a program, thereby supporting an examiner in a case where a subject is imaged by a magnetic resonance imaging apparatus.

### 2. Description of the Related Art

A magnetic resonance imaging (MRI) apparatus magnetically excites atomic nucleus spins of a subject placed in a static magnetic field with a radio frequency (RF) pulse at a Larmor frequency and reconstructs an image from a nuclear magnetic resonance (NMR) signal generated by the excitation. In a case where MR imaging of a patient (subject) is performed by the MRI apparatus, after the patient is laid on an examination table, an examiner (medical staff member) such as a doctor or a technician mounts an instrument to be used for the MR imaging on the patient or disposes the instrument on the examination table. Examples of such an instrument include a receive coil used for the MR imaging, a fixing tool that fixes the patient not to move, and a mat disposed on the examination table.

JP2015-100578A discloses a method of creating recommended measurement conditions for the MRI apparatus based on a specific absorption rate coefficient and body information of the patient. In addition, JP2005-304940A discloses that the MRI apparatus recognizes a type of the receive coil and displays, on a monitor, a mounting method of the receive coil, a method of wiring of the receive coil, and precautions for MR imaging to be explained to the patient.

### SUMMARY OF THE INVENTION

In the MRI examination, the examiner needs to determine a combination of a plurality of types of instruments (receive coil, fixing tool, and mat) to be used according to an examination content (examination site) of the patient. In this case, it is difficult for the examiner, particularly for an examiner with little experience, to determine which instrument should be used in which combination, and it may take time. JP2015-100578A and JP2005-304940A do not disclose that the combination of the receive coil, the fixing tool, and the mat recommended in the MRI examination is presented to the examiner.

The present invention has been made in view of such circumstances, and an object of the present invention is to provide a magnetic resonance imaging support apparatus, an operation method of a magnetic resonance imaging support apparatus, and a program that present a recommended combination of a receive coil, a fixing tool, and a mat to an examiner.

A first aspect of the present invention relates to a magnetic resonance imaging support apparatus comprising: a processor, in which the processor is configured to: acquire examination information including an examination site of a subject to be imaged by a magnetic resonance imaging apparatus; input the examination information to a learning model that has been trained on a relationship between the examination information and a combination of one or a plurality of receive coils that receive a nuclear magnetic resonance signal generated from the examination site, one or a plurality of mats provided on an examination table on which the subject is disposed, and one or a plurality of fixing tools that fix the subject; acquire the combination corresponding to the examination information from the learning model; and output the combination as presentation information.

With the magnetic resonance imaging support apparatus, it is possible to present the examiner with the recommended combination of the receive coil, the fixing tool, and the mat corresponding to the examination site of the subject.

A first embodiment of the present invention relates to the magnetic resonance imaging support apparatus according to the first aspect, in which the processor is configured to acquire the examination information including at least one of a disease, a body type, a gender, or a body position of the subject, in addition to the examination site. As a result, it is possible to present the examiner with the recommended combination of the receive coil, the fixing tool, and the mat that is more suitable for the patient.

A second embodiment of the present invention relates to the magnetic resonance imaging support apparatus according to the first aspect or first embodiment, in which the processor is configured to: acquire guidance indicating at least one of a mounting position, a mounting method, or a method of use of the receive coil, the mat, and the fixing tool corresponding to the combination, based on the combination acquired from the learning model; and output the presentation information including the combination and the guidance. As a result, even an examiner with little experience can easily discriminate at least one of the mounting position, the mounting method, or the method of use of the instrument corresponding to the combination.

A third embodiment of the present invention relates to the magnetic resonance imaging support apparatus according to any one of the first aspect or first to second embodiments, in which the processor is configured to: refer to instrument information in which usable receive coils, mats, and fixing tools are registered; and output, as the presentation information, the combination of usable receive coils, mats, and fixing tools corresponding to the examination information, based on the instrument information. As a result, since the presentation information including an unusable instrument is prevented from being presented to the examiner, it is possible to present the examiner with more appropriate presentation information.

A fourth embodiment of the present invention relates to the magnetic resonance imaging support apparatus according to the third embodiment, in which the processor is configured to acquire information on the usable receive coils, mats, and fixing tools to update the instrument information. As a result, latest information on the usable instruments is reflected in the instrument information.

A fifth embodiment of the present invention relates to the magnetic resonance imaging support apparatus according to any one of the first aspect or first to fourth embodiments, in which the processor is configured to: acquire combination information indicating the combination of the receive coil, the mat, and the fixing tool used for imaging by the magnetic resonance imaging apparatus; and perform training of the learning model using the examination information of the subject imaged by the magnetic resonance imaging apparatus and the combination information as training data. As a result, it is possible to improve the accuracy of the learning model.

A sixth embodiment of the present invention relates to the magnetic resonance imaging support apparatus according to any one of the first aspect or first to fifth embodiments, in which the processor is configured to output the presentation information to a presentation device.

A seventh embodiment of the present invention relates to the magnetic resonance imaging support apparatus according to the sixth embodiment, in which the processor is configured to output the presentation information to at least one of a monitor provided in the magnetic resonance imaging apparatus, a projection device that projects an image onto a wall surface of a shielded room in which the magnetic resonance imaging apparatus is installed, or a monitor held by a flexible arm, as the presentation device.

An operation method of a magnetic resonance imaging support apparatus according to another aspect of the present invention, comprises: via a computer functioning as the magnetic resonance imaging support apparatus, executing a step of acquiring examination information including an examination site of a subject to be imaged by a magnetic resonance imaging apparatus; a step of inputting the examination information to a learning model that has been trained on a relationship between the examination information and a combination of one or a plurality of receive coils that receive a nuclear magnetic resonance signal generated from the examination site, one or a plurality of mats provided on an examination table on which the subject is disposed, and one or a plurality of fixing tools that fix the subject; a step of acquiring the combination corresponding to the examination information from the learning model; and a step of outputting the combination as presentation information.

A program according to still another aspect of the present invention causes a computer functioning as a magnetic resonance imaging support apparatus to implement: a function of acquiring examination information including an examination site of a subject to be imaged by a magnetic resonance imaging apparatus; a function of inputting the examination information to a learning model that has been trained on a relationship between the examination information and a combination of one or a plurality of receive coils that receive a nuclear magnetic resonance signal generated from the examination site, one or a plurality of mats provided on an examination table on which the subject is disposed, and one or a plurality of fixing tools that fix the subject; a function of acquiring the combination corresponding to the examination information from the learning model; and a function of outputting the combination as presentation information.

The present invention can present a recommended combination of a receive coil, a fixing tool, and a mat to an examiner.

### BRIEF DESCRIPTION OF THE DRAWINGS

FIG. 1 is a schematic diagram of an MRI system that executes an MRI examination of a patient.
FIG. 2 is a functional block diagram showing a function of a processor in a case of presenting a recommended combination of a receive coil, a fixing tool, and a mat to an examiner.
FIG. 3 is a diagram showing an example of an input screen of examination information according to a first embodiment displayed on a monitor by an examination information input unit.
FIG. 4 is an explanatory diagram for describing an example of a learning model according to the first embodiment.
FIG. 5 is a diagram showing another example of combination information output by the learning model.
FIG. 6 is a diagram showing an example of training data used for machine learning of the learning model according to the first embodiment.
FIG. 7 is a diagram showing an example of presentation information displayed on a gantry monitor before or during examination preparation work.
FIG. 8 is a diagram showing an example of highlighting an instrument designated by an examiner in an illustration in a list being displayed on a gantry monitor.
FIG. 9 is a flowchart showing a flow of presentation processing of presentation information to an examiner in the MRI system according to the first embodiment.
FIG. 10 is a diagram showing an example of an input screen 221 of examination information according to a second embodiment displayed on a monitor by an examination information input unit.
FIG. 11 is an explanatory diagram for describing an example of a learning model according to the second embodiment.
FIG. 12 is a diagram showing an example of training data used for machine learning of the learning model according to the second embodiment.
FIG. 13 is a functional block diagram of a processor of a console constituting an MRI system according to a third embodiment.
FIG. 14 is an explanatory diagram for describing training of a learning model by a data learning unit.
FIG. 15 is a flowchart showing a flow of training (retraining) of the learning model by the data learning unit.
FIG. 16 is a functional block diagram of a processor of a console constituting an MRI system according to a fourth embodiment.
FIG. 17 is a diagram showing an example of instrument information.
FIG. 18 is a diagram for describing output of combination information by a trained data analysis unit according to the fourth embodiment.
FIG. 19 is a flowchart showing a flow of presentation processing of presentation information to an examiner in the MRI system according to the fourth embodiment.
FIG. 20 is a schematic diagram of an MRI system according to a fifth embodiment.
FIG. 21 is a schematic diagram of an MRI system according to a sixth embodiment.

### DESCRIPTION OF THE PREFERRED EMBODIMENTS

### First embodiment

### Overall configuration of MRI system 10

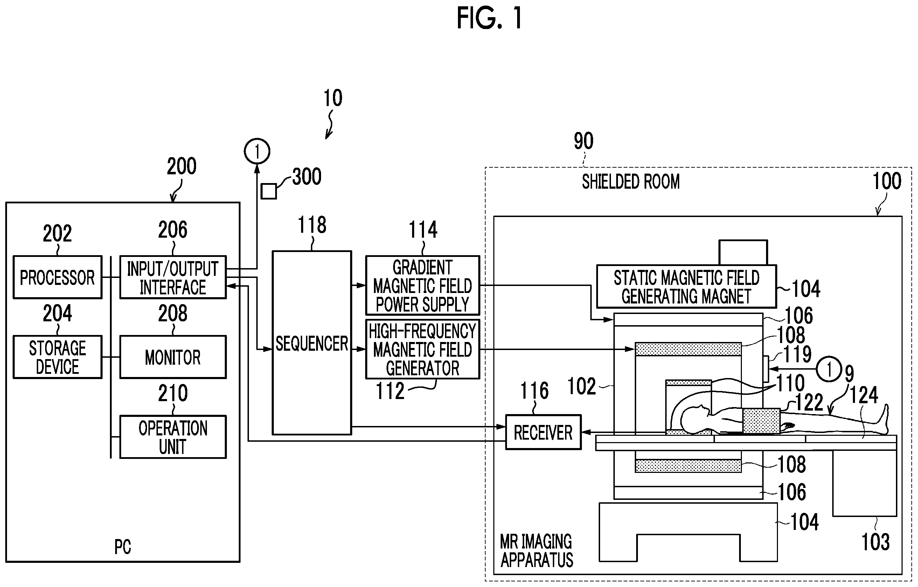

FIG. 1 is a schematic diagram of an MRI system 10 that executes an MRI examination of a patient 9 corresponding to a subject according to an embodiment of the present invention. The MRI system 10 comprises an MR imaging apparatus 100 provided in a shielded room 90 that is an examination room, a high-frequency magnetic field generator 112, a gradient magnetic field power supply 114, a sequencer 118, and a personal computer (PC) 200 provided outside the shielded room 90. The MR imaging apparatus 100 corresponds to a magnetic resonance imaging apparatus according to the embodiment of the present invention, and the PC 200 corresponds to a magnetic resonance imaging support apparatus (a computer that functions as a magnetic resonance imaging support apparatus) according to the embodiment of the present invention.

The MR imaging apparatus 100 executes MR imaging of the patient 9 under the control of the PC 200. The MR imaging apparatus 100 comprises a gantry 102, an examination table 103, a static magnetic field generating magnet 104, a gradient magnetic field coil 106, a radio frequency (RF) coil 108, a receive coil 110, a receiver 116, and a gantry monitor 119.

The gantry 102 has a cylindrical imaging space called a bore. The gantry 102 is provided with the static magnetic field generating magnet 104, the gradient magnetic field coil 106, the RF coil 108, the gantry monitor 119, and the like. The patient 9 to be imaged by the MR imaging apparatus 100 is usually disposed in the imaging space in the gantry 102 in a state of lying on the examination table 103. The examination table 103 (also referred to as a table) is fixed to the gantry 102, but a movable dockable examination table that can be attached to and detached from the gantry 102 may be used as the examination table 103.

The static magnetic field generating magnet 104 generates a uniform static magnetic field in the imaging space (hereinafter, simply referred to as an "imaging space") in the gantry 102 in which the patient 9 is disposed. The static magnetic field generating magnet 104 includes a static magnetic field generating source of a permanent magnet type, a normal conducting type, or a superconducting type.

The gradient magnetic field coil 106 generates a gradient magnetic field in the imaging space. The gradient magnetic field coil 106 is connected to a gradient magnetic field power supply 114 disposed outside the shielded room 90. The gradient magnetic field coil 106 is composed of an X-axis gradient magnetic field coil, a Y-axis gradient magnetic field coil, and a Z-axis gradient magnetic field coil that generate gradient magnetic fields Gx, Gy, and Gz in the X-axis direction, the Y-axis direction, and the Z-axis direction of the imaging space, respectively, and generates a gradient magnetic field corresponding to a current input from the gradient magnetic field power supply 114 in the imaging space. Usually, in a coordinate system of three orthogonal axes of the imaging space, a direction of a central axis of the gantry 102 is defined as a Z-axis direction, a vertical direction is defined as a Y-axis direction, and a direction orthogonal to each of the Z-axis and the Y-axis is defined as an X-axis direction.

The Z-axis gradient magnetic field coil generates the gradient magnetic field Gz for selecting a slice position and a slice width of a plane of the patient 9 orthogonal to the Z-axis direction. The X-axis gradient magnetic field coil generates the gradient magnetic field Gx proportional to a position in the X-axis direction as a gradient magnetic field in a read-out direction (frequency encoding direction) during a period in which an echo signal is generated. The Y-axis gradient magnetic field coil generates the gradient magnetic field Gy in the phase encoding direction having different intensities for each repetition time.

The RF coil 108 (also referred to as a transmitting coil) is connected to a high-frequency magnetic field generator 112 disposed outside the shielded room 90. A high-frequency current pulse having a Larmor frequency that causes nuclear magnetic resonance is input to the RF coil 108 from the high-frequency magnetic field generator 112. The RF coil 108 generates an RF pulse (high-frequency magnetic field) that causes an NMR signal (nuclear magnetic resonance signal) to be generated in an atomic nucleus of an atom constituting a tissue of the patient 9 based on the high-frequency current pulse input from the high-frequency magnetic field generator 112.

The receive coil 110 (also referred to as an RF probe) is mounted on the patient 9 and detects the NMR signal generated from the patient 9 in response to the RF pulse generated from the RF coil 108. The receive coil 110 amplifies the detected NMR signal with a preamplifier (not shown) and then transmits the amplified NMR signal to the receiver 116.

It should be noted that, in FIG. 1, the receive coil 110 used for MR imaging of the head of the patient 9 is shown as an example, but the receive coil 110 corresponding to an examination site is used according to the examination site (chest, abdomen, waist, shoulder, limbs, or the like) of the patient 9. In addition, the receive coil 110 may be a blanket type that is placed over the chest, abdomen, and waist of the patient 9.

A fixing tool 122 for fixing the patient 9 to prevent movement is mounted on the patient 9 as necessary, and a mat 124 for the patient 9 to lie on is disposed on the examination table 103 as necessary.

The receiver 116 includes an amplification amplifier, an analog-to-digital (A/D) converter, and other signal processing circuits. The receiver 116 performs A/D conversion and necessary signal processing on the NMR signal transmitted from the receive coil 110 to generate digital data (also referred to as reception signal data or measurement data). The receiver 116 outputs the generated digital data to the PC 200.

The sequencer 118 is disposed outside the shielded room 90 and is connected to the high-frequency magnetic field generator 112, the gradient magnetic field power supply 114, and the receiver 116. The sequencer 118 transmits control information to the high-frequency magnetic field generator 112 and the gradient magnetic field power supply 114 in accordance with a pulse sequence (imaging sequence) in which an RF pulse, gradient magnetic fields Gx, Gy, and Gz, and the timing and intensity of signal reception are described. As a result, a high-frequency current pulse is input from the high-frequency magnetic field generator 112 to the RF coil 108, and a current is input from the gradient magnetic field power supply 114 to the gradient magnetic field coil 106 in accordance with the control information. It should be noted that various pulse sequences are known depending on the purpose, and examples thereof include a spin echo method, a gradient echo method, and various pulse sequences derived from these.

In addition, the sequencer 118 controls the receiver 116 by transmitting the timing of the A/D conversion performed by the receiver 116 (sampling timing in the frequency encoding direction), information on the amplifier gain, and the like to the receiver 116.

The gantry monitor 119 corresponds to a presentation device according to the embodiment of the present invention, and is, for example, a touch panel type display in which a liquid crystal display or an organic electro-luminescence (EL) display and an operation unit are integrally formed. The gantry monitor 119 is disposed, for example, on at least one of the left or right side of the bore of the front surface of the gantry 102, or is disposed directly above the bore. The examination content of the patient 9 is displayed on the gantry monitor 119. In addition, as will be described in detail later, the gantry monitor 119 displays a recommended combination of "one or a plurality of receive coils 110", "one or a plurality of fixing tools 122", and "one or a plurality of mats 124" (hereinafter, simply referred to as "receive coil 110, fixing tool 122, and mat 124") to be used for the MRI examination of the patient 9.

The PC 200 executes control of the MR imaging by the MR imaging apparatus 100 and display control of the gantry monitor 119. The sequencer 118, the receiver 116, the gantry monitor 119, and the like are communicably connected to the PC 200 via wired connection or wireless connection. As a result, the receive coil 110, the sequencer 118, and the gantry monitor 119 can communicate with the PC 200.

In addition, the PC 200 performs various types of operations such as image reconstruction based on the NMR signal obtained from the MR imaging apparatus 100. Further, the PC 200 determines the recommended combination of the receive coil 110, the fixing tool 122, and the mat 124 to be used for the MRI examination of the patient 9, outputs the presentation information 300 including the combination to the gantry monitor 119, and displays the presentation information 300 on the gantry monitor 119. The PC 200 may be a workstation or a server computer.

The PC 200 comprises, as hardware, a processor 202, a storage device 204, an input/output interface 206, a monitor 208, an operation unit 210, and the like.

The processor 202 may be configured by one or a plurality of pieces of hardware, and the type of hardware is not limited. For example, the processor 202 may be configured by hardware such as a central processing unit (CPU), a micro processing unit (MPU), a programmable logic device such as a field programmable gate array (FPGA), a dedicated circuit for executing specific processing, such as an application-specific integrated circuit (ASIC), a graphic processing unit (GPU), or a neural processing unit (NPU). In addition, the processor 202 includes each unit or each means for executing various types of processing in the present embodiment. Additionally, the type of hardware may be a combination of different types of hardware. In a case where a plurality of hardware components are configured to execute one or a plurality of processes of a certain processor 202, the plurality of hardware components may be present in devices physically separate from each other or may be present within the same device. Further, in any of the embodiments, the order of each process performed by the processor 202 is not limited to the order described above, and may be changed as appropriate. The hardware is configured by an electrical circuit (circuitry) in which circuit elements such as semiconductor elements are combined.

Further, the present embodiment may be implemented by hardware, software, firmware, microcode, or a combination thereof. Software, firmware, and microcode are configured by a program. In addition, the program may be, for example, a program module group, and each function thereof may be implemented by a processor 202 configured to execute each function. The program may be a program code or a plurality of code segments stored in one or a plurality of non-transitory computer-readable media (for example, storage media, other storages, or the like). The program may be divided and stored in the plurality of non-transitory computer-readable media present in devices physically separate from each other. The program code or code segment may represent any combination of a procedure, a function, a subprogram, a routine, a subroutine, a module, a software package, a class, or an instruction, a data structure, or a program statement. The program code or code segment may be connected to another code segment or a hardware circuit by transmitting and receiving information, data, an argument, a parameter, or a content of a memory.

The processor 202 performs overall control of each unit of the PC 200 and the MR imaging apparatus 100, and executes various programs stored in the storage device 204, thereby implementing various functions including control of MR imaging by the MR imaging apparatus 100, image reconstruction of the MR image, display control of the gantry monitor 119, and output of the presentation information 300.

The storage device 204 includes a memory and a storage. The memory includes one or more of a random access memory (RAM), a flash memory, and a read-only memory (ROM). The memory functions as a work area for processing by the processor 202, temporarily stores a program or the like stored in the storage, or temporarily stores digital data input from the MR imaging apparatus 100. The processor 202 may incorporate a part (RAM) of the storage device 204.

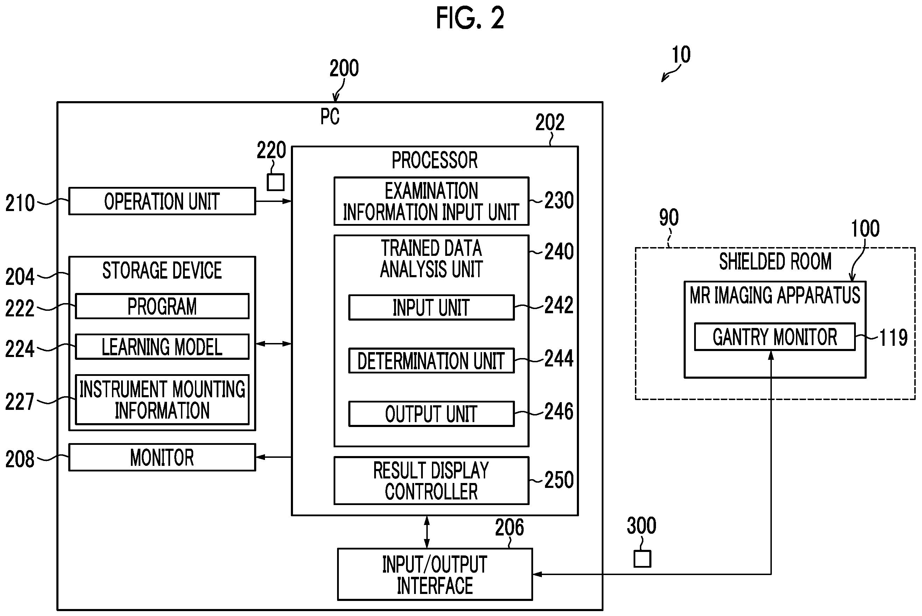

The storage includes, for example, one or more of a hard disk apparatus, a solid-state drive apparatus, and a removable recording medium. The storage stores a program 222 (see FIG. 2) that causes the processor 202 to function as the PC 200, various pulse sequences, a calculation formula used for image reconstruction, a parameter, a learning model 224 (see FIG. 2) described later, instrument mounting information 227 (see FIG. 2) described later, an MR image obtained by image reconstruction, and the like. A part of the data stored in the storage may be stored in another server.

The input/output interface 206 includes a communication unit that can be connected to a network and a connection unit that can be connected to an external apparatus. Various types of data are transmitted and received between the processor 202, the sequencer 118, the receiver 116, the gantry monitor 119, and the like via the input/output interface 206.

The monitor 208 is composed of, for example, a liquid crystal display, an organic electro-luminescence (OEL) display, a projector, or an appropriate combination thereof. Various types of information are displayed on the monitor 208 in addition to the MR image. The monitor 208 is also used as a part of a user interface (UI) in a case of receiving an input from the operation unit 210. The number of the monitors 208 is not limited to one, and a multi-display comprising a plurality of display devices can also be used.

The operation unit 210 includes an input device such as a mouse and a keyboard. The operation unit 210 functions as a part of a graphical user interface (GUI) that receives an input from an examiner for an operation window using the operation window displayed on the monitor 208. For example, the operation unit 210 and the monitor 208 function as a GUI for the examiner to input the start-up, stop (temporary stop), selection of the pulse sequence, the imaging conditions, the processing conditions, and the like of the MR imaging apparatus 100. The operation unit 210 may include a voice input device. In addition, the operation unit 210 may be a touch panel type input device that is integrally configured with a display screen of the monitor 208.

### Function of processor 202

FIG. 2 is a functional block diagram showing a function of the processor 202 in a case of presenting a recommended combination of the receive coil 110, the fixing tool 122, and the mat 124 to the examiner (displaying presentation information 300). Since a control function of the MR imaging apparatus 100 (control function of MR imaging) and an image reconstruction function of the MR image by the processor 202 are known technologies, the illustration thereof is not shown in the drawings.

As shown in FIG. 2, the processor 202 determines a recommended combination of the receive coil 110, the fixing tool 122, and the mat 124 before or during the examination preparation work (hereinafter, abbreviated as "examination preparation work") in which the examiner mounts the receive coil 110 and the fixing tool 122 to the patient 9 or disposes the mat 124 on the examination table 103.

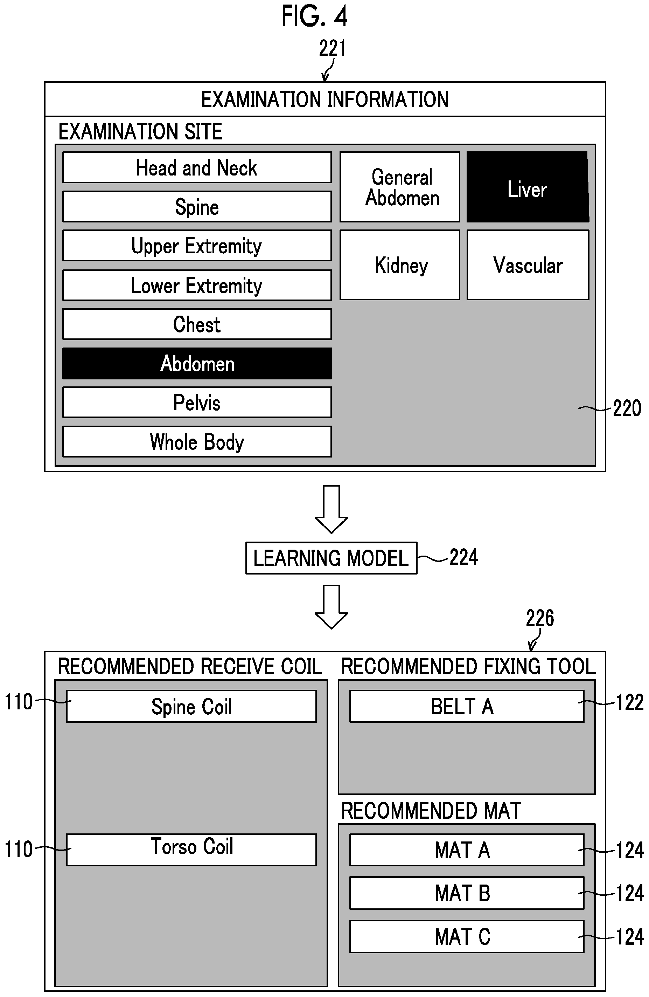

Specifically, the processor 202 determines the recommended combination of the receive coil 110, the fixing tool 122, and the mat 124 by using a learning model 224 based on examination information 220 input to the operation unit 210 by the examiner. Then, the processor 202 generates and outputs the presentation information 300 to be presented to the examiner based on combination information 226 (see FIG. 4) indicating the combination. In this case, the processor 202 functions as an examination information input unit 230, a trained data analysis unit 240, and a result display controller 250 by executing the program 222 read out from the storage device 204.

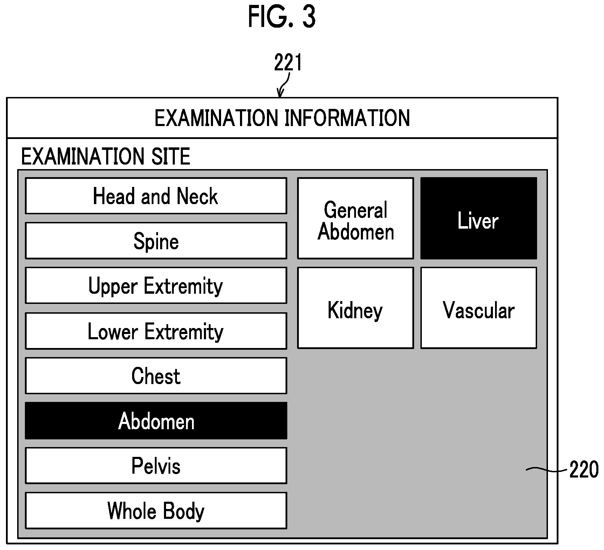

FIG. 3 is a diagram showing an example of an input screen 221 of the examination information 220 according to the first embodiment displayed on the monitor 208 by the examination information input unit 230. As shown in FIG. 3, the examination information input unit 230 displays the input screen 221 of the examination information 220 of the patient 9 on the monitor 208. The examination information 220 according to the first embodiment is information indicating an examination site of the MRI examination of the patient 9. Therefore, the input screen 221 includes selection fields for a plurality of examination sites. For example, in the present embodiment, the input screen 221 includes a human body part selection field for selecting a human body part and an examination site selection field for selecting an examination site corresponding to the human body part selected in the human body part selection field. As a result, the examiner can execute a selection operation of selecting the examination site of the MRI examination of the patient 9 by operating the operation unit 210 (or by performing a touch operation on the screen of the monitor 208) based on the input screen 221 displayed on the monitor 208.

In a case where the examiner inputs the selection operation of the examination site to the operation unit 210, for example, the selection fields of the human body part and the examination site in the input screen 221 selected by the selection operation are switched from white display to black display. It should be noted that, as long as the selection fields (human body part and examination site) selected by the selection operation can be discriminated, a method of switching the display mode of the selection fields is not particularly limited, and any method can be adopted.

Returning to FIG. 2, in a case where the examiner inputs the selection operation of the examination site to the operation unit 210, the examination information 220 indicating the examination site of the MRI examination of the patient 9 is input from the operation unit 210 to the examination information input unit 230. As a result, the examination information input unit 230 acquires the examination information 220 and outputs the examination information 220 to the trained data analysis unit 240.

The trained data analysis unit 240 is a so-called artificial intelligence (AI) processing circuit. The trained data analysis unit 240 outputs combination information 226 (see FIG. 4) indicating a recommended combination of the receive coil 110, the fixing tool 122, and the mat 124 corresponding to the examination site of the patient 9 by using the learning model 224 based on the examination information 220 input from the examination information input unit 230. It should be noted that the learning model 224 has been trained on a relationship between the "examination information 220" and the "recommended combination of the receive coil 110, the fixing tool 122, and the mat 124 corresponding to the examination site" in advance through machine learning.

FIG. 4 is an explanatory diagram for describing an example of the learning model 224 (also referred to as a trained model or a machine learning model) according to the first embodiment. As shown in FIG. 4, the learning model 224 outputs the combination information 226 indicating the recommended combination of the receive coil 110, the fixing tool 122, and the mat 124 corresponding to the examination site, which is a response variable, by using the examination site of the MRI examination, which is the examination information 220, as input data (input variable).

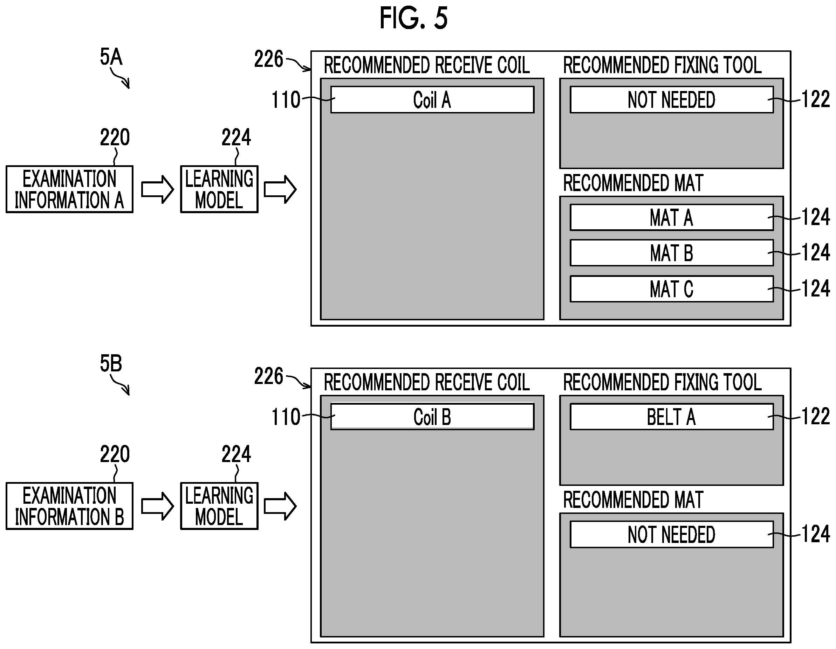

FIG. 5 is a diagram showing another example of the combination information 226 output by the learning model 224. Depending on the examination site of the patient 9, it is not necessary to use all three types of instruments of the receive coil 110, the fixing tool 122, and the mat 124, and the fixing tool 122 may not be needed, the mat 124 may not be needed, or both the fixing tool 122 and the mat 124 may not be needed. Therefore, the combination information 226 output by the learning model 224 includes a case where the fixing tool 122 is "not needed" as shown in an upper part (see reference numeral 5A) of FIG. 5 and a case where the mat 124 is "not needed" as shown in a lower part (see reference numeral 5B) of FIG. 5. That is, the recommended combination of the receive coil 110, the fixing tool 122, and the mat 124 in the present embodiment includes a combination in which the fixing tool 122 is "not needed", a combination in which the mat 124 is "not needed", and a combination in which both the fixing tool 122 and the mat 124 are "not needed".

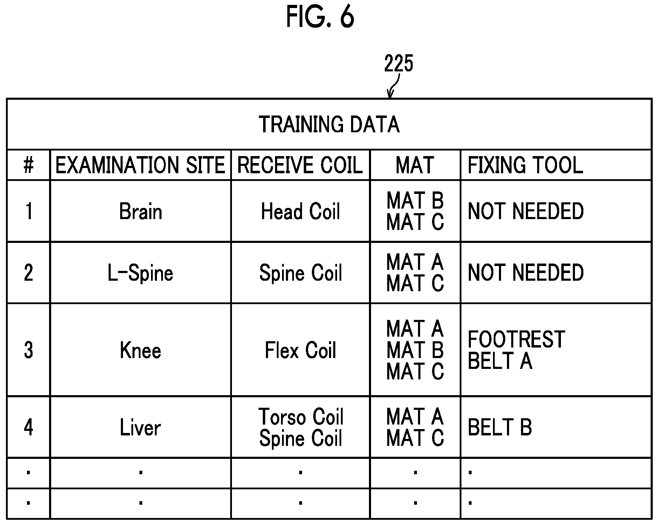

FIG. 6 is a diagram showing an example of training data 225 (also referred to as training data or learning data) used for the machine learning of the learning model 224 according to the first embodiment. As shown in FIG. 6, in the training data 225, the recommended combination (correct answer label) of the receive coil 110, the fixing tool 122, and the mat 124 is associated with each type of the examination site of the MRI examination. The learning model 224 is generated by constructing, for example, a recommendation system (collaborative filtering) using supervised learning with a "k-nearest neighbor method" or a "neural network" based on such training data 225. A method of generating the learning model 224 (machine learning algorithm) is not particularly limited, and any method can be adopted.

Returning to FIG. 2, the trained data analysis unit 240 functions as, for example, an input unit 242, a determination unit 244, and an output unit 246. The determination unit 244 reads out and stores the learning model 224 in advance from the storage device 204.

The input unit 242 inputs the examination information 220 input from the examination information input unit 230 to the determination unit 244. The determination unit 244 determines the recommended combination of the receive coil 110, the fixing tool 122, and the mat 124 by inputting the examination information 220 to the learning model 224. The output unit 246 outputs the determination result of the determination unit 244 as the combination information 226. A configuration of the learning model 224 is not particularly limited as long as the combination information 226 can be output with the examination information 220 as input data.

The result display controller 250 acquires the combination information 226 output from the output unit 246. In addition, the result display controller 250 refers to the instrument mounting information 227 stored in the storage device 204.

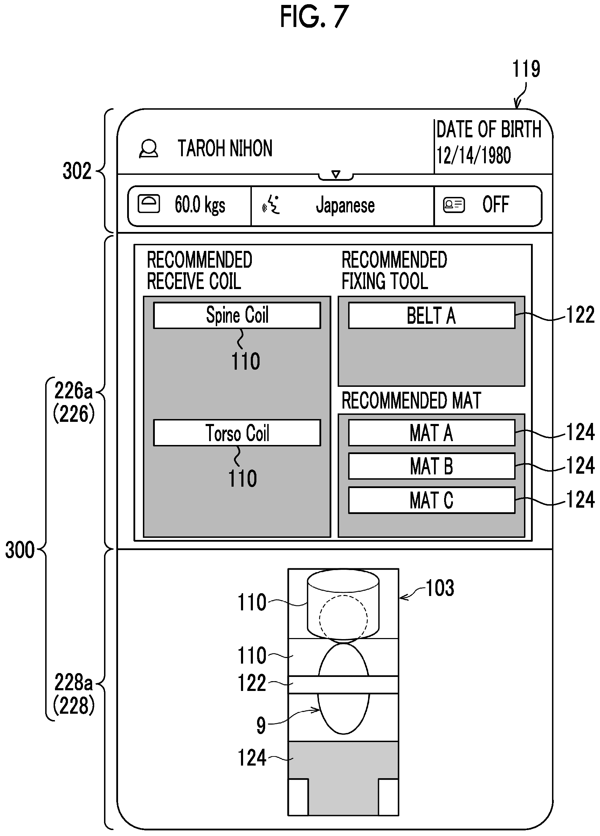

The instrument mounting information 227 is information in which the mounting position, the mounting method, the method of use, and the like are stored for each type of the receive coil 110, each type of the fixing tool 122, and each type of the mat 124. Then, the result display controller 250 acquires the guidance 228 (see FIG. 7) indicating the mounting position, the mounting method, and the method of use of the receive coil 110, the fixing tool 122, and the mat 124 corresponding to the combination information 226 by referring to the instrument mounting information 227. The guidance 228 need only include at least one of the mounting position, the mounting method, or the method of use of the instrument.

Next, the result display controller 250 generates the presentation information 300 to be presented to the examiner based on the combination information 226 and the guidance 228. The presentation information 300 may be configured with only the combination information 226, but in this case, the examiner with little experience may feel a psychological burden of not knowing the mounting position, the mounting method, and the method of use of the receive coil 110, the fixing tool 122, and the mat 124. Therefore, it is preferable that the presentation information 300 includes the guidance 228.

The result display controller 250 outputs the presentation information 300 to the gantry monitor 119 via the input/output interface 206. As a result, the presentation information 300 is displayed on the gantry monitor 119.

FIG. 7 is a diagram showing an example of the presentation information 300 displayed on the gantry monitor 119 before or during the examination preparation work.

As shown in FIG. 7, the presentation information 300 and patient information 302 are displayed on the gantry monitor 119 before or during the examination preparation work. The patient information 302 includes, for example, information such as a name and a date of birth of the patient 9 who is the target of the MRI examination. The patient information 302 is not particularly limited as long as it is information related to the patient 9.

The presentation information 300 displayed on the gantry monitor 119 includes, for example, a list 226a corresponding to the combination information 226 and an illustration 228a corresponding to the guidance 228.

For example, the list 226a displays instrument names of all the receive coils 110, the fixing tools 122, and the mats 124 included in the combination information 226. The display mode of the combination information 226 is not limited to the list 226a, and can be changed as appropriate.

The illustration 228a is, for example, an image in which the appearance, the mounting position, the mounting method, the method of use, and the like of the receive coil 110, the fixing tool 122, and the mat 124 included in the combination information 226 are visualized. The image of the patient 9 in the illustration 228a may not be shown in the drawings. In addition, the illustration 228a may include descriptions of the mounting positions, the mounting methods, and the methods of use of the receive coil 110, the fixing tool 122, and the mat 124.

The display mode of the guidance 228 is not limited to the illustration 228a, and can be changed as appropriate. For example, the display mode is not particularly limited as long as the mounting positions, the mounting methods, and the methods of use of the receive coil 110, the fixing tool 122, and the mat 124 can be discriminated, and may be, for example, only the description.

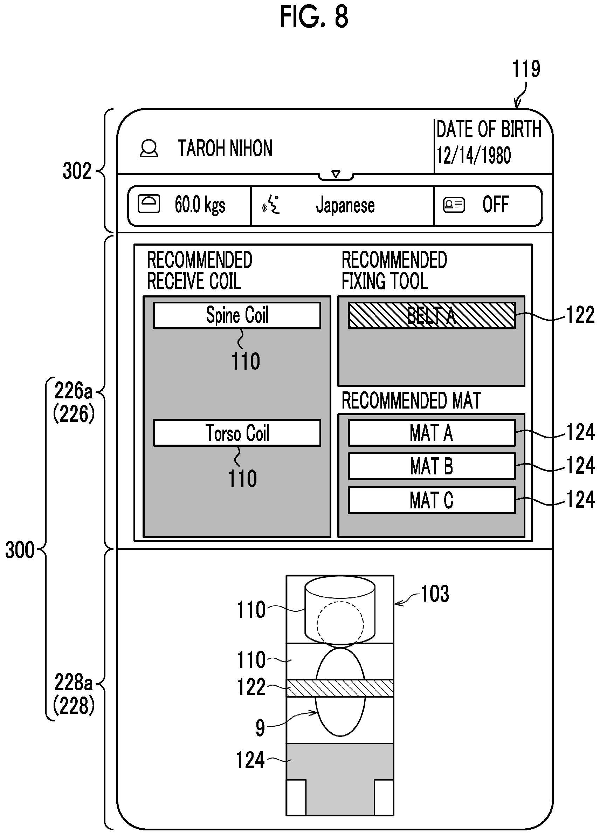

FIG. 8 is a diagram showing an example in which an instrument designated by the examiner in the list 226a being displayed on the gantry monitor 119 is highlighted in the illustration 228a. As shown in FIG. 8, in a case where the examiner executes a selection operation, for example, a touch operation on any instrument (here, "mat A") in the list 226a, the processor 202 (result display controller 250) highlights the image of the instrument (mat A) selected by the selection operation and the mounting position thereof in the illustration 228a.

### Action of MRI system 10 according to first embodiment

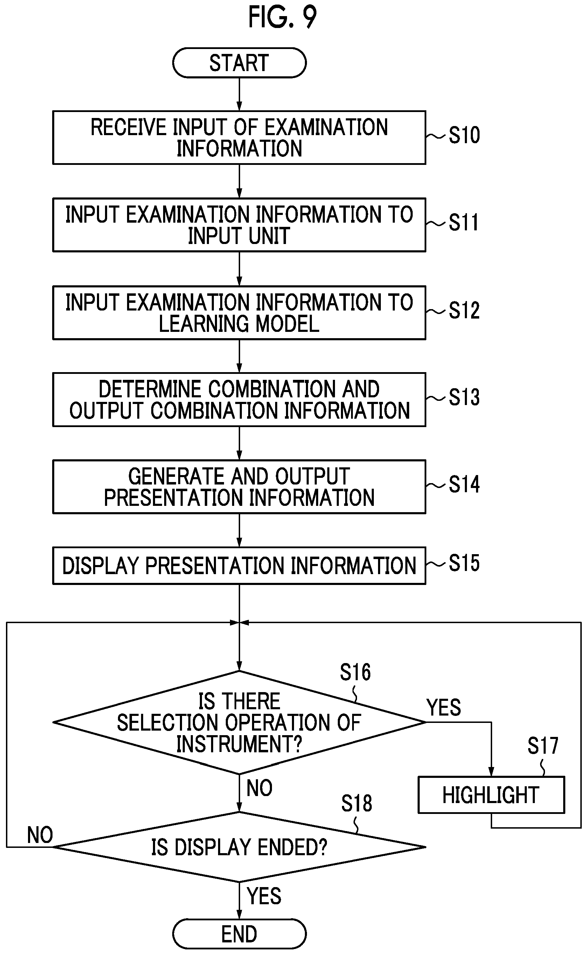

FIG. 9 is a flowchart showing a flow of presentation processing of the presentation information 300 to the examiner (an operation method of the magnetic resonance imaging support apparatus according to the embodiment of the present invention) in the MRI system 10 according to the first embodiment having the above-described configuration.

As shown in FIG. 9, before or during the examination preparation work, the examination information input unit 230 displays the input screen 221 on the monitor 208 in response to a display operation by the examiner, or automatically displays the input screen 221 on the monitor 208. Then, as shown in FIG. 3 described above, in a case where the examiner executes the selection operation of the examination site of the patient 9 by operating the operation unit 210 while viewing the input screen 221, the examination information input unit 230 acquires the examination information 220 by receiving the input of the examination information 220 from the operation unit 210, and outputs the examination information 220 to the trained data analysis unit 240 (step S10). As a result, the examination information 220 is input to the input unit 242 of the trained data analysis unit 240 (step S11).

Next, the input unit 242 inputs the examination information 220 to the determination unit 244, and the determination unit 244 inputs the examination information 220 to the learning model 224 (step S12). As a result, as shown in FIGS. 4 and 5 described above, the determination unit 244 determines the recommended combination of the receive coil 110, the fixing tool 122, and the mat 124 corresponding to the examination site of the patient 9 based on the output result of the learning model 224 (step S13). Then, the output unit 246 outputs the determination result of the determination unit 244 to the result display controller 250 as the combination information 226.

In a case where the result display controller 250 acquires the combination information 226 from the output unit 246, the result display controller 250 acquires the guidance 228 corresponding to the receive coil 110, the fixing tool 122, and the mat 124 included in the combination information 226 by referring to the instrument mounting information 227. Then, the result display controller 250 generates the presentation information 300 based on the combination information 226 and the guidance 228, and outputs the presentation information 300 to the gantry monitor 119 via the input/output interface 206 (step S14).

The gantry monitor 119 displays the presentation information 300 input from the input/output interface 206 (step S15). As a result, as shown in FIG. 7 described above, the examiner can recognize the recommended combination of the receive coil 110, the fixing tool 122, and the mat 124 corresponding to the examination site of the patient 9 based on the list 226a corresponding to the combination information 226.

In addition, the examiner can discriminate the appearance, the mounting position, the mounting method, and the method of use of the receive coil 110, the fixing tool 122, and the mat 124 based on the illustration 228a corresponding to the guidance 228. As a result, even an examiner with little experience can easily discriminate the appearance, the mounting position, the mounting method, and the method of use of the receive coil 110, the fixing tool 122, and the mat 124 recommended in the presentation information 300. In addition, the examiner with little experience does not feel a psychological burden of not knowing the mounting position, the mounting method, and the method of use of the receive coil 110, the fixing tool 122, and the mat 124.

In a case where the examiner executes a selection operation of any instrument in the list 226a after the display of the presentation information 300 by the gantry monitor 119 (YES in step S16), the processor 202 (result display controller 250) highlights the image of the instrument selected by the selection operation and the mounting position or the like of the instrument in the illustration 228a as shown in FIG. 8 described above (step S17). As a result, the examiner can individually and easily discriminate the shape, the mounting position, the mounting method, and the method of use of the receive coil 110, the fixing tool 122, and the mat 124 registered in the list 226a.

Hereinafter, the display of the presentation information 300 is continued until the examiner inputs the display end operation of the presentation information 300 to the screen of the operation unit 210 or the gantry monitor 119 (NO in step S16, NO in step S18), and the display of the presentation information 300 is ended in response to the input of the display end operation (YES in step S18).

As described above, in the MRI system 10 according to the first embodiment, the learning model 224 that outputs the combination of the receive coil 110, the fixing tool 122, and the mat 124 in response to the input of the examination information 220 is used, so that the examiner can be presented with the combination of the receive coil 110, the fixing tool 122, and the mat 124 recommended for the MRI examination of the patient 9.

### Second embodiment

Next, the MRI system 10 according to a second embodiment will be described. In the MRI system 10 according to the first embodiment, the examination information 220 including the information on the examination site of the patient 9 is input to the learning model 224. In this case, it may be desirable to change or limit the combination of the receive coil 110, the fixing tool 122, and the mat 124 used for the MRI examination depending on the body type (height and weight), the disease (case), and the gender of the patient 9. In addition, in a case where the patient 9 can only be in a body position (sleeping position and posture) different from the normal body position, it is desirable to select the combination of the receive coil 110, the fixing tool 122, and the mat 124 corresponding to the body position.

Therefore, in the MRI system 10 according to the second embodiment, the examination information 220 including the information on the examination site of the patient 9 and the information on the patient 9 other than the examination site is input to the learning model 224. The MRI system 10 according to the second embodiment has basically the same configuration as the MRI system 10 according to the first embodiment, except that a part of the examination information 220 input to the learning model 224 is different from that in the first embodiment. Therefore, the same reference numerals are given to those having the same function or configuration as those of the first embodiment, and the description thereof will be omitted.

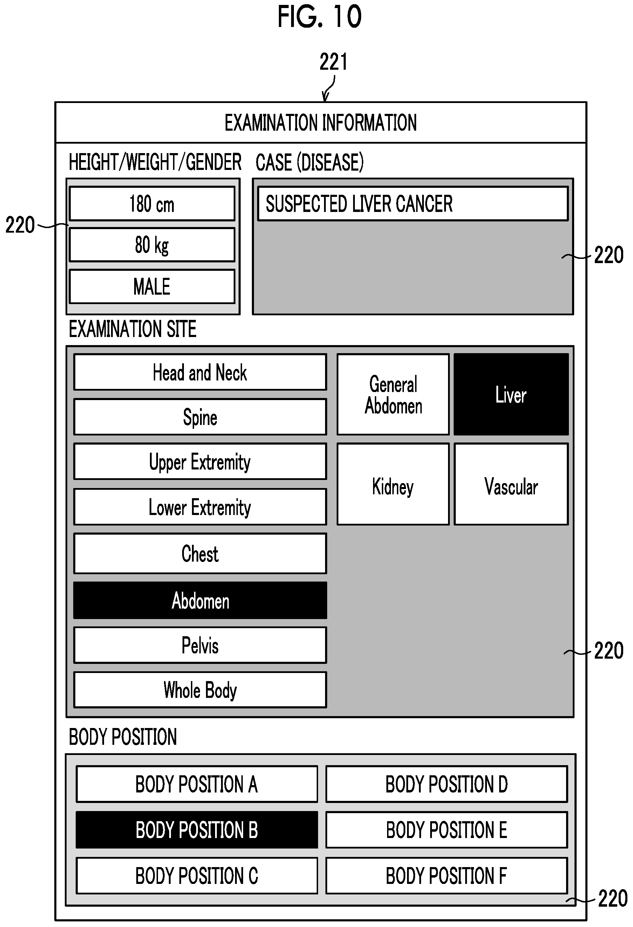

FIG. 10 is a diagram showing an example of an input screen 221 of the examination information 220 according to the second embodiment displayed on the monitor 208 by the examination information input unit 230. As shown in FIG. 10, the examination information 220 according to the second embodiment includes information on the body type (height and weight), the disease (case), the gender, and the body position of the patient 9, in addition to the examination site of the patient 9. It is sufficient that at least one of the information on the body type, the disease (case), the gender, or the body position is included in the examination information 220.

The input screen 221 according to the second embodiment includes an input field for the height, the weight, and the gender of the patient 9, an input field for the case (disease) of the patient 9, and a selection field for the body position that can be taken by the patient 9, in addition to the selection field for the plurality of examination sites. As a result, the examiner can execute the selection operation of selecting the examination site and the body position and the input operation of inputting the height, the weight, the gender, and the case (disease) by operating the operation unit 210 based on the input screen 221 displayed on the monitor 208, as in the first embodiment.

In a case where the examiner executes the selection operation or the input operation of each item (examination site, height, weight, gender, case (disease), and body position) of the input screen 221 on the operation unit 210, the examination information 220 including the information on each of the items is input from the operation unit 210 to the examination information input unit 230. Then, the examination information input unit 230 acquires the examination information 220 and outputs the examination information 220 to the trained data analysis unit 240, as in the first embodiment.

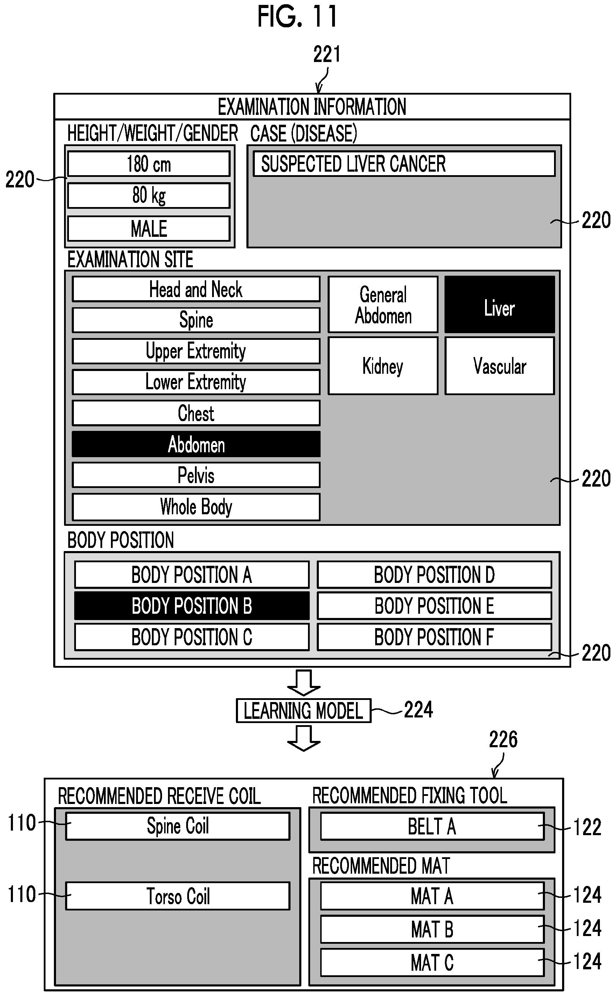

FIG. 11 is an explanatory diagram for describing an example of the learning model 224 according to the second embodiment. As shown in FIG. 11, the learning model 224 according to the second embodiment outputs combination information 226 indicating a recommended combination of the receive coil 110, the fixing tool 122, and the mat 124 corresponding to each item by using each item of the examination site, the height, the weight, the gender, the case (disease), and the body position of the patient 9, which is the examination information 220, as input data.

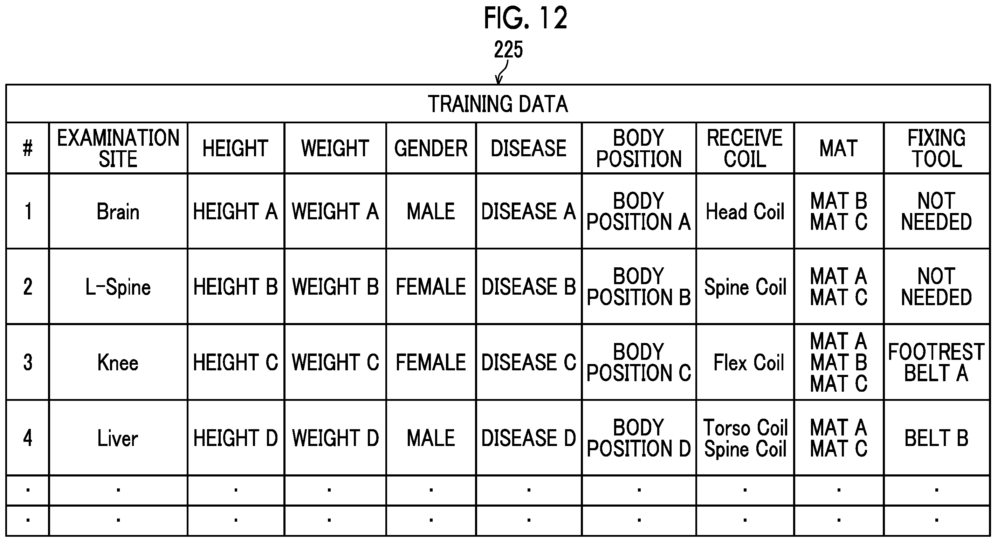

FIG. 12 is a diagram showing an example of training data 225 used for the machine learning of the learning model 224 according to the second embodiment. As shown in FIG. 12, in the training data 225 according to the second embodiment, the recommended combination (correct answer label) of the receive coil 110, the fixing tool 122, and the mat 124 is associated with each combination of the items of the examination site, the height, the weight, the gender, the case (disease), and the body position. Then, the learning model 224 according to the second embodiment is generated by the same method as in the first embodiment based on such training data 225.

The trained data analysis unit 240 according to the second embodiment outputs the combination information 226 indicating the recommended combination of the receive coil 110, the fixing tool 122, and the mat 124 corresponding to each item of the patient 9 by inputting the examination information 220 including the information on each item of the examination site, the height, the weight, the gender, the case (disease), and the body position of the patient 9 to the learning model 224.

It should be noted that a flow of the presentation processing of the presentation information 300 to the examiner in the MRI system 10 according to the second embodiment is basically the same as that in the first embodiment shown in FIG. 9 described above, and thus, the specific description thereof will be omitted here.

As described above, in the MRI system 10 according to the second embodiment, the examiner can be presented with the recommended combination of the receive coil 110, the fixing tool 122, and the mat 124 corresponding to each item of the examination site, the height, the weight, the gender, the case (disease), and the body position of the patient 9. As a result, the examiner can be presented with the recommended combination of the receive coil 110, the fixing tool 122, and the mat 124 that is more suitable for the patient 9 than in the first embodiment. In addition, the examiner can be presented with the recommended combination of the receive coil 110, the fixing tool 122, and the mat 124 corresponding to the patient 9 who can only take a body position different from the normal body position.

### Third embodiment

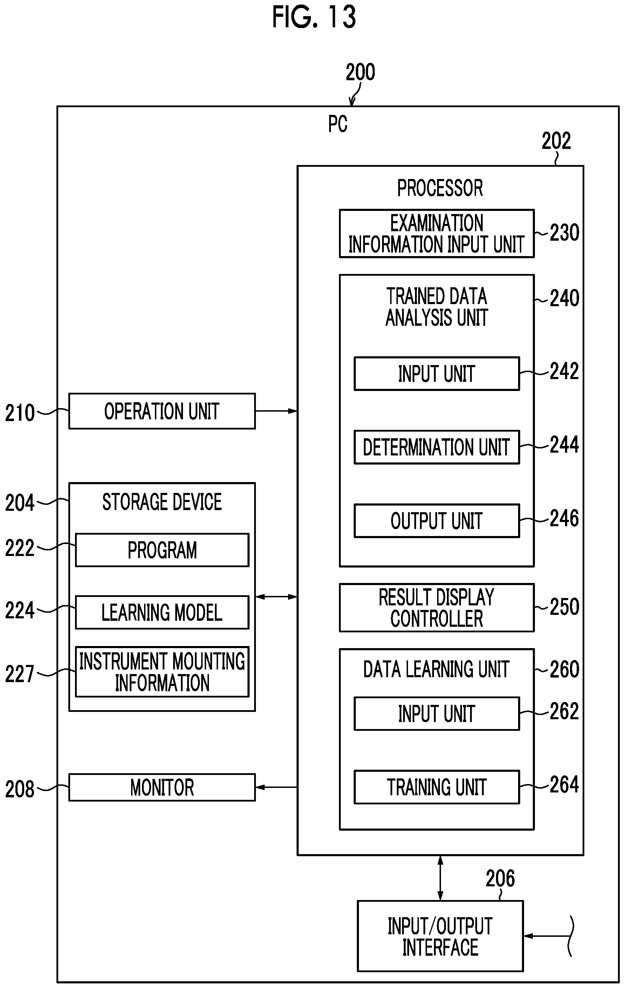

FIG. 13 is a functional block diagram of the processor 202 of the PC 200 constituting the MRI system 10 according to the third embodiment. In the MRI system 10 according to each of the above-described embodiments, the training (retraining, optimization, or additional training) of the learning model 224 is not executed, but in the MRI system 10 according to the third embodiment, the training of the learning model 224 is executed.

As shown in FIG. 13, the MRI system 10 according to the third embodiment has basically the same configuration as the MRI system 10 according to each of the above-described embodiments, except that the processor 202 of the PC 200 also functions as a data learning unit 260. Therefore, the same reference numerals are given to those having the same function or configuration as those of each of the above-described embodiments, and the description thereof will be omitted.

The data learning unit 260 functions as an input unit 262 and a training unit 264.

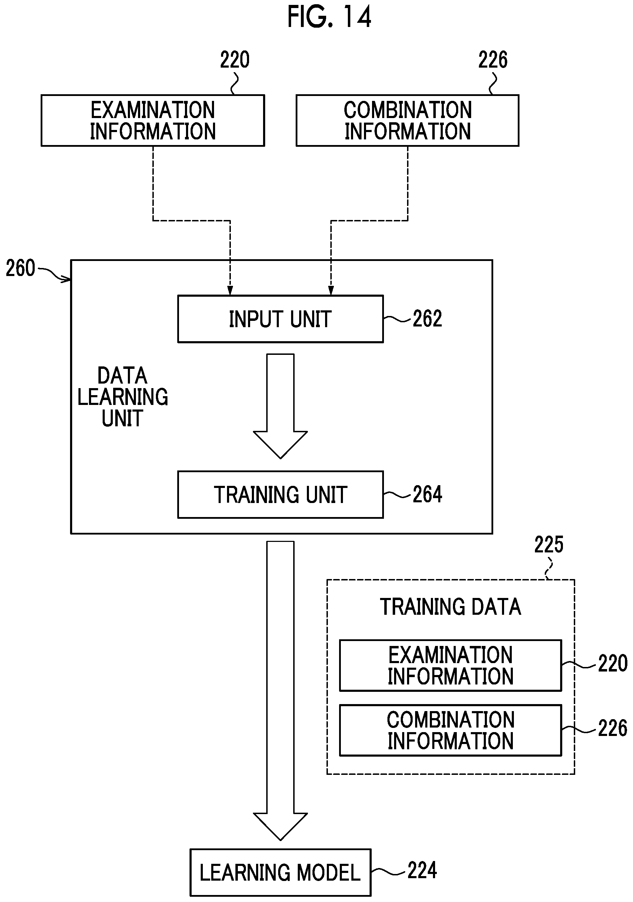

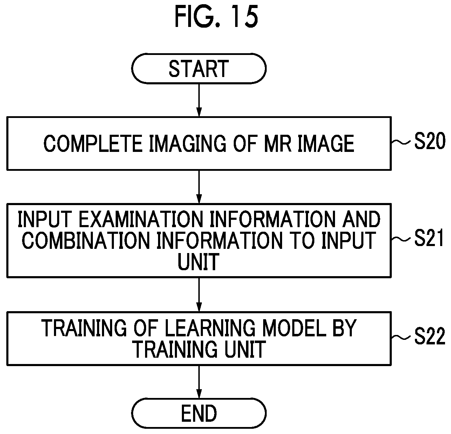

FIG. 14 is an explanatory diagram for describing the training of the learning model 224 by the data learning unit 260. FIG. 15 is a flowchart showing a flow of the training (retraining) of the learning model 224 by the data learning unit 260.

As shown in FIGS. 14 and 15, in a case where the MR imaging by the MR imaging apparatus 100 is completed (step S20 in FIG. 15), the examination information 220 input to the examination information input unit 230 and the combination information 226 of the receive coil 110, the fixing tool 122, and the mat 124 actually used for the MR imaging are input to the input unit 262 (step S21 in FIG. 15).

The examiner may manually input the examination information 220 to the input unit 262 using the operation unit 210. In addition, the combination information 226 may be input from the trained data analysis unit 240 to the input unit 262, or may be manually input to the input unit 262 by the examiner using the operation unit 210.

In addition, the input timing of the examination information 220 and the combination information 226 to the input unit 262 is not limited to the timing of the completion of the MR imaging, and may be, for example, a timing at which the combination information 226 is output from the trained data analysis unit 240 or a timing at which the mounting of the receive coil 110, the fixing tool 122, and the mat 124 is completed by the examiner.

Then, the input unit 262 inputs the examination information 220 and the combination information 226 to the training unit 264.

The training unit 264 generates the training data 225 by associating the examination information 220 and the combination information 226 input from the input unit 262. Next, the training unit 264 executes the training of the learning model 224 by executing the supervised learning described above based on the training data 225 (step S22 in FIG. 15). Hereinafter, the training of the learning model 224 is repeatedly executed each time the MRI examination is executed. As a result, the trained data analysis unit 240 can output the combination information 226 corresponding to the MRI examination of the patient 9 by using the learning model 224 whose accuracy is improved by the training by the data learning unit 260.

As described above, in the MRI system 10 according to the third embodiment, the training data 225 is generated based on the combination information 226 used for the actual MR imaging and the examination information 220 of the patient 9 input before the MR imaging, and the training of the learning model 224 is performed by using the training data 225, so that the accuracy of the learning model 224 can be improved. As a result, it is possible to present the combination of the receive coil 110, the fixing tool 122, and the mat 124 that is more suitable for the MRI examination of the patient 9 to the examiner.

### Fourth embodiment

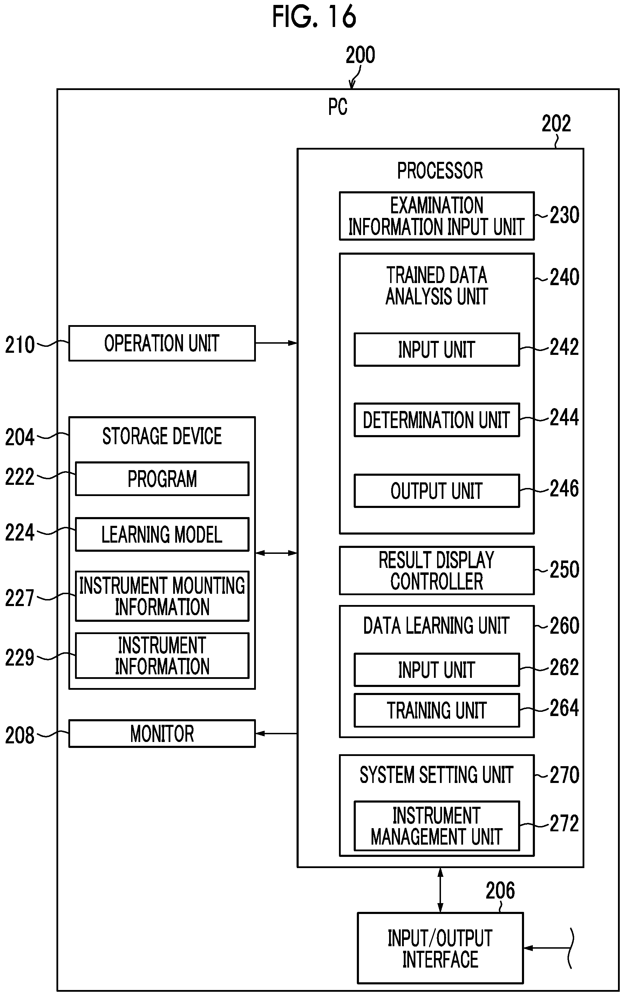

FIG. 16 is a functional block diagram of the processor 202 of the PC 200 constituting the MRI system 10 according to the fourth embodiment. In the MRI system 10 according to each of the above-described embodiments, the recommended combination of the receive coil 110, the fixing tool 122, and the mat 124 corresponding to the MRI examination of the patient 9 is presented to the examiner. In this case, in a medical facility (hereinafter, abbreviated as a medical facility) in which the MRI system 10 is installed, at least one of the receive coil 110, the fixing tool 122, or the mat 124 included in the combination presented by the MRI system 10 may be broken (damaged).

Therefore, in the MRI system 10 according to the fourth embodiment, the combination of the receive coil 110, the fixing tool 122, and the mat 124 that can be used in the medical facility is presented to the examiner.

As shown in FIG. 16, the MRI system 10 according to the fourth embodiment has basically the same configuration as the MRI system 10 according to the third embodiment, except that the processor 202 of the PC 200 also functions as a system setting unit 270 and instrument information 229 is stored in the storage device 204. Therefore, the same reference numerals are given to those having the same function or configuration as those of each of the above-described embodiments, and the description thereof will be omitted. It should be noted that, similarly to the first embodiment or the second embodiment, the data learning unit 260 of the processor 202 can be omitted.

The system setting unit 270 performs system settings for each unit of the MRI system 10. The system setting unit 270 functions as an instrument management unit 272. The instrument management unit 272 registers or deletes an instrument in the instrument information 229 stored in the storage device 204.

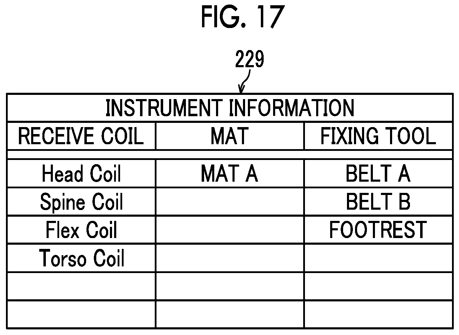

FIG. 17 is a diagram showing an example of the instrument information 229. In the instrument information 229, information (for example, an instrument name) on the receive coil 110, the fixing tool 122, and the mat 124 that are usable in the medical facility (that are not broken or damaged) is registered. The instrument management unit 272 registers usable instruments (the receive coil 110, the fixing tool 122, and the mat 124) in the instrument information 229 in response to a registration operation of the usable instruments for the operation unit 210 by the examiner. It should be noted that the instrument management unit 272 can delete information on an unusable instrument from the instrument information 229 in response to a deletion operation of the unusable instrument for the operation unit 210 by the examiner.

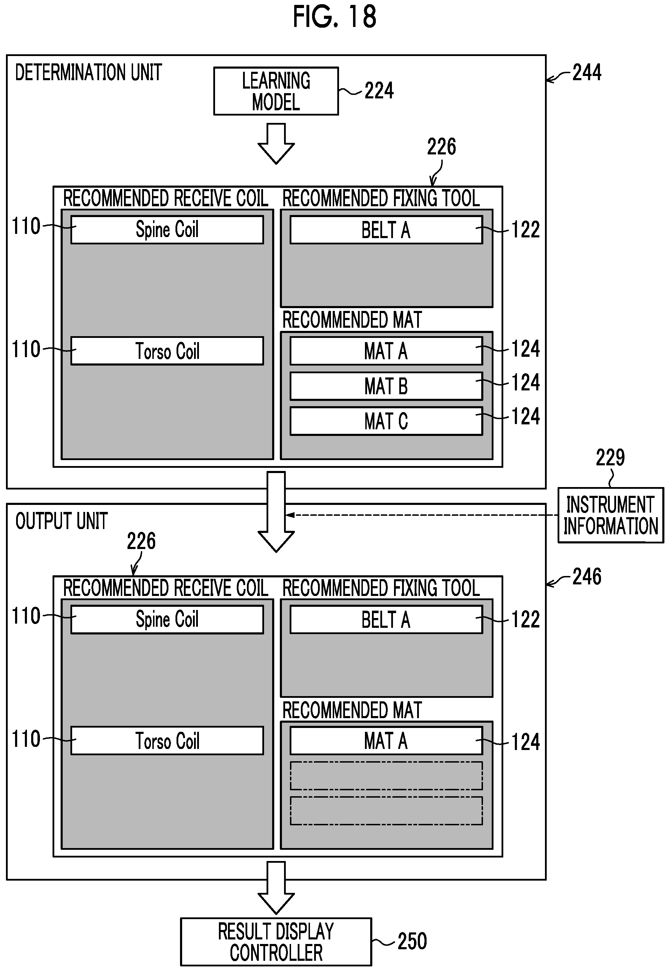

FIG. 18 is a diagram for describing the output of the combination information 226 by the trained data analysis unit 240 (particularly, the determination unit 244 and the output unit 246) according to the fourth embodiment. As shown in FIG. 18, the determination unit 244 according to the fourth embodiment determines a recommended combination of the receive coil 110, the fixing tool 122, and the mat 124 corresponding to the MRI examination of the patient 9 based on the output result from the learning model 224, as in each of the above-described embodiments.

The output unit 246 according to the fourth embodiment compares the combination of the receive coil 110, the fixing tool 122, and the mat 124 determined by the determination unit 244 with the instrument information 229 read out from the storage device 204, and discriminates an instrument that is usable in the medical facility among the instruments (the receive coil 110, the fixing tool 122, and the mat 124) included in the combination determined by the determination unit 244. For example, in the example shown in FIG. 18, it is assumed that the "mat B" and the "mat C" are unusable, but other instruments are usable (see FIG. 17).

Next, the output unit 246 according to the fourth embodiment outputs the combination information 226 indicating the combination of the receive coil 110, the fixing tool 122, and the mat 124 that are usable in the medical facility and are recommended for the MRI examination of the patient 9 to the result display controller 250 based on the discrimination result of the instrument that is usable in the medical facility.

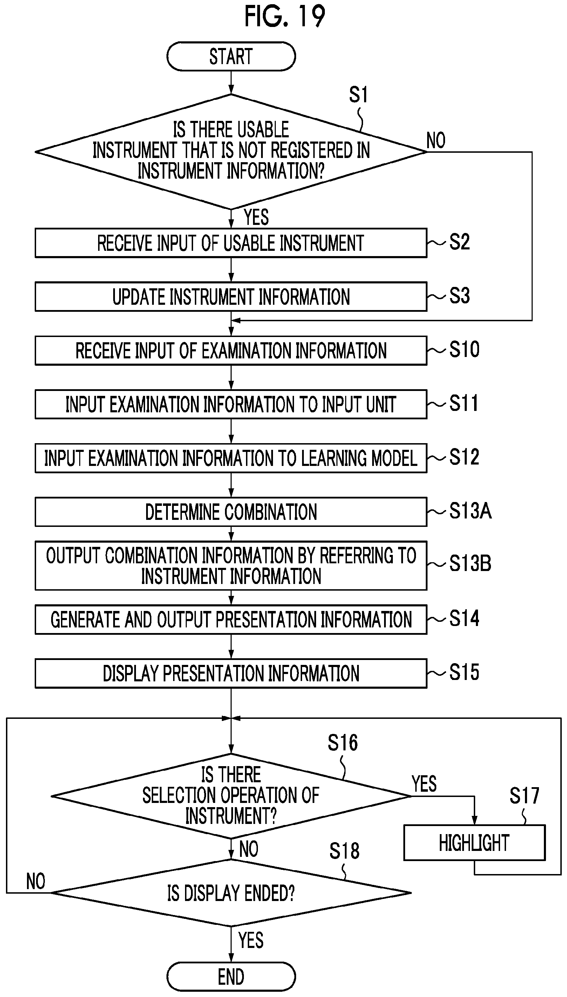

FIG. 19 is a flowchart showing a flow of presentation processing of presentation information 300 to the examiner in the MRI system 10 according to the fourth embodiment. As shown in FIG. 19, at any timing at which the instrument information 229 can be updated, such as after the MR imaging is completed or before the new MR imaging is started, in a case where there is a usable instrument that is not registered in the instrument information 229, the examiner performs a registration operation of the usable instrument on the operation unit 210 (YES in step S1). In a case where there is no usable instrument that is not registered in the instrument information 229, the processing proceeds to step S10 (NO in step S1).

In a case where the examiner executes the registration operation on the operation unit 210, the instrument management unit 272 receives the information on the usable instrument based on the registration operation and updates the instrument information 229 in the storage device 204 (registers the information on the usable instrument) (step S2 and step S3). As a result, the latest information on the instrument that is usable in the medical facility is reflected in the instrument information 229 (step S3).

Although not shown, in a case where a new instrument that is broken or damaged occurs at the timing of step S1, the examiner performs a deletion operation of the unusable instrument on the operation unit 210. In this case, in step S2 to step S3, the instrument management unit 272 receives the information on the unusable instrument and updates the instrument information 229 (deletes the information on the unusable instrument).

Hereinafter, as in the first embodiment described above (see FIG. 9), the processing from step S10 to step S12 is executed, so that the determination unit 244 determines the recommended combination of the receive coil 110, the fixing tool 122, and the mat 124 corresponding to the MRI examination of the patient 9 as shown in FIG. 18 described above (step S13A).

Next, the output unit 246 discriminates the usable instrument among the respective instruments (the receive coil 110, the fixing tool 122, and the mat 124) included in the combination determined by the determination unit 244 with reference to the instrument information 229 read out from the storage device 204. Then, the output unit 246 outputs the combination information 226 indicating the recommended combination of the receive coil 110, the fixing tool 122, and the mat 124 that are usable in the medical facility to the result display controller 250 (step S13B).

Since the subsequent processing is the same as that in the first embodiment described above (see FIG. 9), the specific description thereof will be omitted here.

As described above, in the MRI system 10 according to the fourth embodiment, by registering the usable instrument in the instrument information 229, the trained data analysis unit 240 can output the combination information 226 of the receive coil 110, the fixing tool 122, and the mat 124 corresponding to the MRI examination of the patient 9 after recognizing the usable instrument with reference to the instrument information 229. As a result, since the presentation information 300 including the unusable instrument in the medical facility is prevented from being presented to the examiner, more appropriate presentation information 300 can be presented to the examiner.

### Fifth embodiment

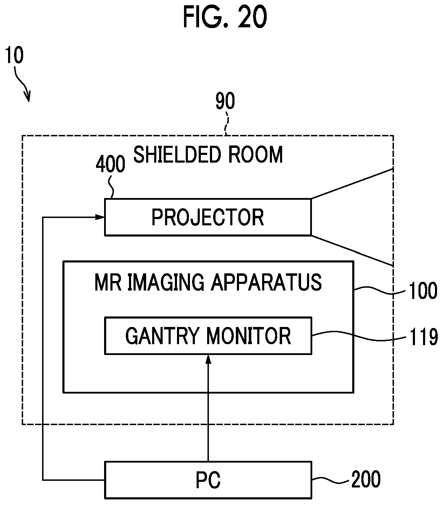

FIG. 20 is a schematic diagram of an MRI system 10 according to a fifth embodiment. In the MRI system 10 according to each of the above-described embodiments, the presentation information 300 is displayed on the gantry monitor 119, but the presentation information 300 may be displayed on another display device.

As shown in FIG. 20, the MRI system 10 according to the fifth embodiment has basically the same configuration as the MRI system 10 according to each of the above-described embodiments, except that the MRI system 10 comprises a projector 400 (also referred to as a projection device) provided in the shielded room 90. Therefore, the same reference numerals are given to those having the same function or configuration as those of each of the above-described embodiments, and the description thereof will be omitted.

The projector 400 corresponds to the presentation device according to the embodiment of the present invention, and displays an image on a wall surface of the shielded room 90 by projecting the image onto the wall surface.

A PC 200 (processor 202) according to the fifth embodiment outputs the presentation information 300 to both the gantry monitor 119 and the projector 400 (or only the projector 400). As a result, the projector 400 displays the presentation information 300 on the wall surface by projecting the presentation information 300 onto the wall surface of the shielded room 90. As a result, the presentation information 300 can be presented to the examiner as in each of the above-described embodiments.

### Sixth embodiment

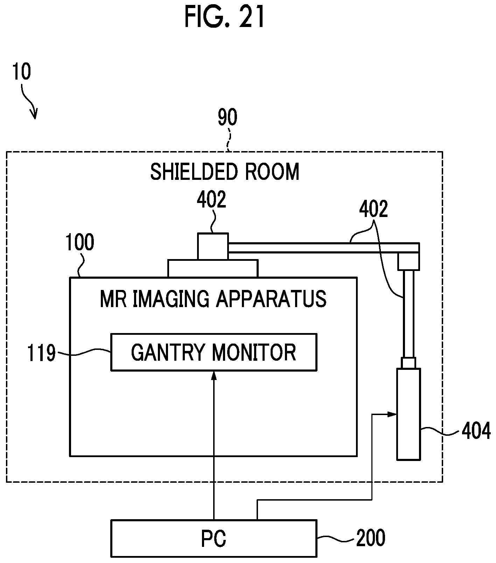

FIG. 21 is a schematic diagram of an MRI system 10 according to a sixth embodiment. In the MRI system 10 according to the fifth embodiment, the presentation information 300 is displayed on the wall surface of the shielded room 90 by the projector 400, but the presentation information 300 may be displayed on another display device.

As shown in FIG. 21, the MRI system 10 according to the sixth embodiment has basically the same configuration as the MRI system 10 according to each of the above-described embodiments, except that the MRI system 10 comprises a flexible arm 402 and a monitor 404. Therefore, the same reference numerals are given to those having the same function or configuration as those of each of the above-described embodiments, and the description thereof will be omitted.

A base end part of the flexible arm 402 is attached to the MR imaging apparatus 100 (or the wall surface, the ceiling, or the floor of the shielded room 90). In addition, the monitor 404 is attached to a tip part of the flexible arm 402. As a result, the flexible arm 402 holds the position and the posture of the monitor 404 to be displaceable.

The monitor 404 corresponds to the presentation device according to the embodiment of the present invention, and is, for example, a liquid crystal display or an organic EL display.

The PC 200 (processor 202) according to the sixth embodiment outputs the presentation information 300 to both the gantry monitor 119 and the monitor 404 (or only the monitor 404). As a result, the monitor 404 displays the presentation information 300. As a result, the presentation information 300 can be presented to the examiner as in each of the above-described embodiments.

### Others

In each of the above-described embodiments, the presentation information 300 is output from the PC 200 to at least one of the gantry monitor 119, the projector 400, or the monitor 404, but the presentation information 300 may be output to another display device (for example, a portable terminal of the examiner). The portable terminal includes a smartphone, a tablet terminal, smart glasses, a head-mounted device, or the like. In addition, the output destination of the presentation information 300 from the PC 200 is not limited to the display device (projection device), and the presentation information 300 (the names of the receive coil 110, the fixing tool 122, and the mat 124, and the like) may be output from the PC 200 to a speaker. In addition, a presentation device other than the speaker may be used.

In each of the above-described embodiments, the PC 200 executes the control of the MR imaging via the MR imaging apparatus 100, the display control of the gantry monitor 119, and the image reconstruction based on the NMR signal, but some of the functions of the PC 200 may be executed by an indoor PC (not shown) disposed in the shielded room 90.

In each of the above-described embodiments, an example has been described in which the PC 200 (processor 202) of the MRI system 10 functions as the magnetic resonance imaging support apparatus according to the embodiment of the present invention, but an indoor PC, an outdoor PC disposed outside the shielded room 90, an in-hospital server of a medical facility, an external cloud server, or the like may function as the magnetic resonance imaging support apparatus according to the embodiment of the present invention. In addition, the functions of the processor of the magnetic resonance imaging support apparatus according to the embodiment of the present invention may be distributed to processors of a plurality of apparatuses (the PC 200, the server, and the like).

In addition, the present invention can also be applied to a program and a program product. The program product includes, for example, a computer-readable medium that is a tangible non-transitory information storage medium on which a program is recorded. In addition, the program can be recorded on a computer-readable medium that is an optical disk, a magnetic disk, a semiconductor memory, or other tangible non-transitory storage medium, and the program can be provided through the storage medium. In addition, instead of the aspect in which the program is stored in the computer-readable medium and provided, it is also possible to provide a program signal as a download service using an electric communication line such as the Internet.

### Explanation of References

9: patient
10: MRI system
90: shielded room
100: MR imaging apparatus
102: gantry
103: examination table
104: static magnetic field generating magnet
106: gradient magnetic field coil
108: RF coil
110: receive coil
112: high-frequency magnetic field generator
114: gradient magnetic field power supply
116: receiver
118: sequencer
119: gantry monitor
122: fixing tool
124: mat
200: PC
202: processor
204: storage device
206: input/output interface
208: monitor
210: operation unit
220: examination information
221: input screen
222: program
224: learning model
225: training data
226: combination information
226a: list
227: instrument mounting information
228: guidance
228a: illustration
229: instrument information
230: examination information input unit
240: trained data analysis unit
242: input unit
244: determination unit
246: output unit
250: result display controller
260: data learning unit
262: input unit
264: training unit
270: system setting unit
272: instrument management unit
300: presentation information
302: patient information
400: projector
402: flexible arm
404: monitor
Gx: gradient magnetic field
Gy: gradient magnetic field
Gz: gradient magnetic field
S1 to S18: presentation processing method of presentation information
S20 and S21: learning method of learning model

## Claims

1. A magnetic resonance imaging support apparatus (200) comprising:
a processor (202),
wherein the processor (202) is configured to:
acquire examination information (220) including an examination site of a subject (9) to be imaged by a magnetic resonance imaging apparatus (100);
input the examination information (220) to a learning model (224) that has been trained on a relationship between the examination information (220) and a combination of one or a plurality of receive coils (110) that receive a nuclear magnetic resonance signal generated from the examination site, one or a plurality of mats (124) provided on an examination table (103) on which the subject (9) is disposed, and one or a plurality of fixing tools (122) that fix the subject (9);
acquire the combination corresponding to the examination information (220) from the learning model (224); and
output the combination as presentation information (300).

2. The magnetic resonance imaging support apparatus (200) according to claim 1,
wherein the processor (202) is configured to acquire the examination information (220) including at least one of a disease, a body type, a gender, or a body position of the subject (9), in addition to the examination site.

3. The magnetic resonance imaging support apparatus (200) according to claim 1 or 2,
wherein the processor (202) is configured to:
acquire guidance (228) indicating at least one of a mounting position, a mounting method, or a method of use of the receive coil (110), the mat (124), and the fixing tool (122) corresponding to the combination, based on the combination acquired from the learning model (224); and
output the presentation information (300) including the combination and the guidance (228).

4. The magnetic resonance imaging support apparatus (200) according to any one of claims 1 to 3,
wherein the processor (202) is configured to:
refer to instrument information (229) in which usable receive coils (110), mats (124), and fixing tools (122) are registered; and
output, as the presentation information (300), the combination of usable receive coils (110), mats (124), and fixing tools (122) corresponding to the examination information (220), based on the instrument information (229).

5. The magnetic resonance imaging support apparatus (200) according to claim 4,
wherein the processor (202) is configured to acquire information on the usable receive coils (110), mats (124), and fixing tools (122) to update the instrument information (229).

6. The magnetic resonance imaging support apparatus (200) according to any one of claims 1 to 5,
wherein the processor (202) is configured to:
acquire combination information (226) indicating the combination of the one or a plurality of receive coils (110), the one or a plurality of mats (124), and the one or a plurality of fixing tools (122) used for imaging by the magnetic resonance imaging apparatus (100); and
perform training of the learning model (224) using the examination information (220) of the subject (9) imaged by the magnetic resonance imaging apparatus (100) and the combination information (226) as training data (225).

7. The magnetic resonance imaging support apparatus (200) according to any one of claims 1 to 5,
wherein the processor (202) is configured to output the presentation information (300) to a presentation device.

8. The magnetic resonance imaging support apparatus (200) according to claim 7,
wherein the processor (202) is configured to output the presentation information (300) to at least one of a monitor (119) provided in the magnetic resonance imaging apparatus (100), a projection device (400) that projects an image onto a wall surface of a shielded room (90) in which the magnetic resonance imaging apparatus (100) is installed, or a monitor (404) held by a flexible arm (402), as the presentation device.

9. An operation method of a magnetic resonance imaging support apparatus (200), the method comprising:
via a computer functioning as the magnetic resonance imaging support apparatus (200), executing
a step (S10) of acquiring examination information (220) including an examination site of a subject (9) to be imaged by a magnetic resonance imaging apparatus (100);
a step (S12) of inputting the examination information (220) to a learning model (224) that has been trained on a relationship between the examination information (220) and a combination of one or a plurality of receive coils (110) that receive a nuclear magnetic resonance signal generated from the examination site, one or a plurality of mats (124) provided on an examination table (103) on which the subject (9) is disposed, and one or a plurality of fixing tools (122) that fix the subject (9);
a step (S13) of acquiring the combination corresponding to the examination information (220) from the learning model (224); and
a step (S14) of outputting the combination as presentation information (300).

10. A non-transitory, computer-readable recording medium on which a program is recorded, the program causing a computer to execute an operation method of a magnetic resonance imaging support apparatus (200) according to claim 9.
